Europäisches Patentamt

(19) European Patent Office

Office européen des brevets

(11) Veröffentlichungsnummer: **0 049 319**
**B1**

(12) **EUROPÄISCHE PATENTSCHRIFT**

(45) Veröffentlichungstag der Patentschrift:
**14.12.83**

(21) Anmeldenummer: **81103302.6**

(22) Anmeldetag: **02.05.81**

(51) Int. Cl.³: **A 61 L 15/01**

(54) **Wundkompresse.**

(30) Priorität: **30.09.80 DE 3036814**

(43) Veröffentlichungstag der Anmeldung:
**14.04.82 Patentblatt 82/15**

(45) Bekanntmachung des Hinweises auf die Patenterteilung:
**14.12.83 Patentblatt 83/50**

(84) Benannte Vertragsstaaten:
**AT BE CH FR GB IT LI LU NL SE**

(56) Entgegenhaltungen:
**GB - A - 1 242 717**
**GB - A - 1 527 592**

(73) Patentinhaber: **Firma Carl Freudenberg, Höhnerweg 2, D-6940 Weinheim/Bergstrasse (DE)**

(72) Erfinder: **Stein, Karl Heinz, Gutenbergstrasse 36, D-6944 Hemsbach (DE)**
Erfinder: **Krull, Manfred, Hüttengasse 49, D-6901 Heiligkreuzsteinach (DE)**

(74) Vertreter: **Weissenfeld-Richters, Helga, Dr., Höhnerweg 2, D-6940 Weinheim/Bergstrasse (DE)**

Wundkompresse

Die Erfindung betrifft eine Wundkompresse, bestehend aus einer Saugschicht und einer damit verbundenen, flüssigkeitsdurchlässigen Abdeckschicht aus hydrophoben Fasern.

Eine Wundkompresse dieser Art ist aus DE-GM 77 03 897 bekannt. Die Abdeckschicht besteht aus einem in sich verfilzten Vliesstoff aus Polypropylenfasern, aus Polyäthylen-, Polyester- und/oder Polyamidfasern. Die Abdeckschicht ist durch eine Hitzekalandrierung mit der Saugschicht verbunden, woraus ein papierartig steifer Griff resultiert. Eine Wundkompresse dieser Art vermag sich deshalb nur in unzureichendem Masse an die Oberfläche einer Wunde anzuschmiegen, und das austretende Wundsekret wird deshalb nicht in allen Bereichen gleich gut aufgenommen. Insbesondere zähflüssige Sekrete führen sehr leicht zu einer Verklebung der Oberfläche, woraus eine weitere Beeinträchtigung der Saugfähigkeit resultieren kann.

Die britische Patentschrift 15 27 592 zeigt eine Ausführung, bei der die Abdeckschicht unabhängig von der Saugschicht erzeugt und anschliessend ohne feste flächige Verbindung lediglich über deren Oberfläche positioniert wird. Dabei ergibt sich die Schwierigkeit, dass die einschlägig verwendeten Saugschichten von der Oberfläche abstehende, freie Faserenden und -schlaufen aufweisen. Diese wirken beim Aufbringen der Abdeckschicht als Abstandhalter, d.h. zwischen beiden Schichten kommt es zur Ausbildung eines Spaltes, der den Flüssigkeitstransport durch die Abdeckschicht und in die Saugschicht hinein in erheblichem Masse beeinträchtigt.

Die frei von der Oberfläche der Saugschicht abstehenden Faserenden und Faserschlaufen werden durch die Abdeckschicht bei einer entsprechenden Ausführung nicht eingebunden. Einzelne Faserenden und Faserschlaufen durchdringen daher die offenen Poren der Abdeckschicht, und können mit der Wunde verkleben.

Der Erfindung liegt die Aufgabe zugrunde, eine besonders weiche und anschmiegsame Wundkompresse mit glatter, fusselfreier Oberfläche zu entwickeln, die alle im medizinischen Bereich praktisch vorkommenden Sekretarten mit ausgezeichneter Wirksamkeit aufnimmt, und die ein Verkleben mit der Wunde während des Heilungsprozesses mit grosser Sicherheit verhindert.

Diese Aufgabe wird erfindungsgemäss mit einer Wundkompresse der eingangs genannten Art gelöst, die dadurch gekennzeichnet ist, dass

a) die Abdeckschicht ein Flächengewicht von 10 bis 50 g/m$^2$ aufweist,

b) die Fasern aus einem aliphatischen Polyurethan bestehen, und einen Durchmesser von 10 bis 40 μm aufweisen,

c) die Abdeckschicht nach Glättung der Saugschicht unmittelbar anschliessend auf die Saugschicht aufgebracht und unter Beibehaltung der Identität der Fasern mit der Saugschicht verklebt ist.

Eine Abdeckschicht dieser Art ist ausgezeichnet mit wässrigen Medien benetzbar, wodurch auf die Oberfläche auftreffende Wundsekrete schnell in die Saugschicht abgeleitet werden. Die Abdeckschicht selbst nimmt keine Feuchtigkeit auf, und ihre gute Durchlässigkeit für Luft- und Wundsekrete bleibt dadurch auch nach längerem Gebrauch voll erhalten. Die gute Anschmiegsamkeit und Weichheit in Verbindung mit den vorgenannten Vorteilen macht die vorgeschlagene Wundkompresse besonders geeignet für Langzeitverbände, die beispielsweise für die Behandlung von Brandwunden benötigt werden.

Die vorgeschlagene Wundkompresse weist eine Temperaturbeständigkeit bis ca. 180 °C auf. Sie kann deshalb in üblicher Weise bei einer Temperatur von ca. 130 °C sterilisiert und entsprechend verwendet werden.

Da die Abdeckschicht ein Flächengewicht von 10 bis 50 g/m$^2$ aufweist, und aus Fasern mit einem Durchmesser von 15 bis 40 μm besteht, ist sie einerseits ausserordentlich dünn und andererseits sehr grob strukturiert. Überraschenderweise wird dennoch eine Oberfläche erhalten, die vollkommen frei von hervorstehenden Fusseln der Saugschicht ist, und die ein Verkleben mit der Wunde während des Heilungsprozesses mit grosser Sicherheit verhindert.

Die Polyurethanfasern der Abdeckschicht sind vorzugsweise autogen untereinander verschweisst, d.h. unter Vermeidung der Hinzuführung irgendwelcher Fremdstoffe, die einen nachteiligen Einfluss auf den Heilungsprozess zur Folge haben könnten. Die Fasern können einander in einer wirren Verteilung zugeordnet werden, d.h. ohne eine bevorzugte Faserlagerung in irgendeiner Richtung. Ihr Profil ist bevorzugt kreisförmig begrenzt. Es ist zwar ebenfalls möglich, gegebenenfalls Fasern mit einem abweichenden Profil zu verwenden, dadurch ergibt sich aber in allen Fällen eine Beeinträchtigung der Durchlässigkeit gegenüber hochviskosen Sekreten. Der Verwendung von Fasern mit einem kreisförmigen begrenzten Profil wird deshalb in allen Fällen der Vorzug gegeben.

Die Polyurethanfasern können eine endliche Länge haben, beispielsweise eine Länge von 4 bis 80 cm. Derartige Fasern lassen sich unter Anwendung einer Sprühspinn-Technologie erzeugen, beispielsweise durch elektrostatische Verspinnung eines gelösten aliphatischen Polyurethans. Bei der Anwendung eines solchen Spinnverfahrens ergibt sich eine Anreicherung der Oberfläche der einzelnen Fasern mit Mikroporen, in die gegebenenfalls die Wundheilung fördernde sekundäre Substanzen eingelagert werden können.

Im Sinne der vorliegenden Erfindungen wird demgegenüber die Verwendung einer Abdeckschicht bevorzugt, die aus endlosen Fasern aufgebaut ist, wie unter Anwendung der bekannten Schmelzspinn-Technologien erhältlich. Die Oberfläche solcher Art erzeugter Fasern ist vollkommen porenfrei und glatt, wodurch die Einlagerung von Wundsekret und dadurch ein Verkleben mit der Wunde noch sicherer vermieden wird. Das vorgeschriebene Profil und der Durchmesser der Fasern sowie die gegenseitige Zuordnung lassen sich sehr exakt steuern, wodurch die Herstellung entsprechender Abdeckschichten unproblematisch ist. Ein weiterer wesentlicher Vorteil besteht darin, dass Abdeckschichten dieser Art herstellungsbedingt keine Fremdstoffe enthalten, die sich nachteilig auf den Heilungsprozess auswirken können.

Die Abdeckschicht und die Saugschicht können in selbständigen Arbeitsstufen hergestellt und anschliessend untereinander verbunden werden. Wegen des ausserordentlich geringen Flächengewichtes weist die Abdeckschicht nur eine geringe Dimensions- und Formstabilität auf, so dass in diesem Falle besondere Vorkehrungen getroffen werden müssen, um eine gleichmässige Kaschierung beider Schichten zu gewährleisten.

Gemäss der vorliegenden Erfindung wird demgegenüber ein Verfahren bevorzugt, bei dem die Saugschicht geglättet wird und bei dem die Polyurethanfasern unmittelbar darauf aufgesponnen werden, wobei die Verfahrensbedingungen so eingestellt werden, dass die Fasern während ihrer Ablage und unter Beibehaltung ihrer Identität untereinander und mit der Saugschicht verkleben.

Die Saugschicht kann in üblicher Weise aus Zellulosefasern bestehen, und sie kann beispielsweise eine Schichtdicke von 3 bis 5 mm haben. Sie wird in einem beheizten Kalander geglättet, der aus einer Stahl- und einer Baumwollwalze besteht, die bei einer Temperatur von 140 °C mit einem Liniendruck von 35 kg/cm gegeneinander angestellt sind. Dei Arbeitsgeschwindigkeit beträgt 25 m/min. Die Saugschicht weist anschliessend eine hochgradig glatte Oberfläche auf, auf die die Fasern der Abdeckschicht in einem Abstand von 10 bis 20 cm aufgesponnen werden. Die Fasern sind während ihrer Ablage noch klebrig, woraus eine gegenseitige Verbindung untereinander und mit der Saugschicht resultiert. Ausserdem ergibt sich eine Stabilisierung der Oberflächen-Struktur der Saugschicht, d.h. die in dem vorausgegangenen Kalandervorgang erzeugte Oberflächenglätte bleibt auch nach der Abkühlung weitgehend erhalten. Die Oberfläche der so erhaltenen Wundkompresse ist vollkommen frei von Fusseln und vorstehenden Faserenden. Für übliche Anwendungsfälle ist deshalb eine weitere Nachbehandlung nicht erforderlich. Nur wenn die Wundkompresse in der plastischen Chirurgie verwendet werden soll, kann es vorteilhaft sein, einen weiteren Glättungsvorgang anzuschliessen, beispielsweise durch eine Nachbehandlung in einem Kalander der vorstehend angesprochenen Art, dessen Walzen bei einem Liniendruck von 20 kp/cm und einer Arbeitsgeschwindigkeit von 20 m/min auf eine Temperatur von 80 °C erwärmt sind.

## Patentansprüche

1. Wundkompresse, bestehend aus einer Saugschicht und einer damit verbundenen, flüssigkeitsdurchlässigen Abdeckschicht aus hydrophoben Fasern, dadurch gekennzeichnet, dass

a) die Abdeckschicht ein Flächengewicht von 10 bis 50 g/m² aufweist,

b) die Fasern aus einem aliphatischen Polyurethan bestehen, und einen Durchmesser von 10 bis 40 μm aufweisen,

c) die Abdeckschicht nach Glättung der Saugschicht unmittelbar anschliessend auf die Saugschicht aufgebracht und unter Beibehaltung der Identität der Fasern mit der Saugschicht verklebt ist.

2. Wundkompresse nach Anspruch 1, dadurch gekennzeichnet, dass die Abdeckschicht aus auf die geglättete Saugschicht derart aufgesponnenen Fasern besteht, dass sie bei einer regellosen Ablage und unter Beibehaltung ihrer Identität untereinander und mit der Saugschicht verkleben.

3. Wundkompresse nach Anspruch 1 bis 2, dadurch gekennzeichnet, dass die Fasern ein kreisförmig begrenztes Profil haben.

4. Wundkompresse nach Anspruch 1 bis 3, dadurch gekennzeichnet, dass die Fasern endlos sind.

5. Wundkompresse nach Anspruch 1 bis 4, dadurch gekennzeichnet, dass die Fasern Sprühspinnfasern sind.

6. Wundkompresse nach Anspruch 1 bis 5, dadurch gekennzeichnet, dass die Fasern Schmelzspinnfasern sind.

7. Wundkompresse nach Anspruch 1 bis 6, dadurch gekennzeichnet, dass die aus den Fasern gebildete Abdeckschicht geglättet ist.

## Revendications

1. Compresse pour blessure, constituée d'une couche absorbante et d'une couche de couverture, en fibres hydrophobes, perméable aux liquides et qui lui est liée, caractérisée en ce que

a) la couche de couverture présente un poids spécifique de surface de 10 à 50 g/m²,

b) les fibres sont constituées de polyuréthane aliphatique d'un diamètre de 10 à 40 microns,

c) la couche de couverture est rapportée directement sur la couche absorbante après avoir rendue lisse celle-ci et collée avec la couche absorbante conservant l'identité des fibres.

2. Compresse pour blessure selon la revendication 1, caractérisée en ce que la couche de couverture est constituée de fibres déposées directement par filage sur la couche absorbante étant réglés de façon que les fibres collent l'une à l'autre et avec la couche absorbante lors d'un dépôt désordonné et en conservant leur identité.

3. Compresse selon les revendications 1–2, caractérisée en ce que les fibres ont un profil limité par une forme circulaire.

4. Compresse selon les revendications 1–3, caractérisée en ce que les fibres sont sans fin.

5. Compresse selon les revendications 1–4, caractérisée en ce que les fibres sont produits par un procédé de filage à projection.

6. Compresse selon les revendications 1–5, caractérisée en ce que les fibres sont produites par filage à chaud.

7. Compresse selon les revendications 1–6, caractérisée en ce que la couche de couverture constituée de fibres est rendue lisse.

## Claims

1. Surgical compress consisting of an absorptive layer connected to a liquid-permeable cover layer of hydrophobic fibers, characterized in that

a) the cover layer has an area weight of 10–50 g/m$^2$,

b) the fibers consist of an aliphatic polyurethane having a diameter of 10 to 40 μm,

c) the cover layer is, after having smoothed the absorptive layer, applied directly onto the absorptive layer and retaining the identity of the fibers bonded to the absorptive layer.

2. Surgical compress according to claim 1, characterized in that the cover layer consists of fibers being spun thus onto the smoothed absorptive layer that they adhere in random arrangement and retaining their identity to each other and to the absorptive layer.

3. Surgical compress according to claims 1–2, characterized in that the fibers have a circular, limited cross section.

4. Surgical compress according to claims 1–3, characterized in that the fibers are endless.

5. Surgical compress according to claims 1–4, characterized in that the fibers are spray-spun fibers.

6. Surgical compress according to claims 1–5, characterized in that the fibers are spun in an extrusion spinning process.

7. Surgical compress according to claims 1–6, characterized in that the cover layer formed by the fibers is smoothed.